(19) Europäisches Patentamt / European Patent Office / Office européen des brevets

(11) **EP 2 606 817 B1**

(12) **FASCICULE DE BREVET EUROPEEN**

(45) Date de publication et mention
de la délivrance du brevet:
**05.12.2018 Bulletin 2018/49**

(51) Int Cl.:
*A61B 5/00* *(2006.01)*    *G01N 21/47* *(2006.01)*
*G01N 21/64* *(2006.01)*

(21) Numéro de dépôt: **12198720.0**

(22) Date de dépôt: **20.12.2012**

(54) **Système de reconstruction de propriétés optiques d'un milieu diffusant, comprenant une source de rayonnement pulsée et au moins deux détecteurs de deux types différents, et procédé de reconstruction associé**

System zur Wiederherstellung der optischen Eigenschaften eines Diffusionsmediums, das eine pulsierende Strahlungsquelle und mindestens zwei Detektoren unterschiedlichen Typs umfasst, und entsprechendes Wiederherstellungsverfahren

System for reconstructing optical properties of a scattering medium, including a source of pulsed radiation and at least two detectors of two different types, and associated reconstruction method

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **23.12.2011 FR 1162412**

(43) Date de publication de la demande:
**26.06.2013 Bulletin 2013/26**

(73) Titulaire: **Commissariat à l'Énergie Atomique et aux Énergies Alternatives**
**75015 Paris (FR)**

(72) Inventeurs:
• **Dinten, Jean-Marc**
  **69008 Lyon (FR)**
• **Boutet, Jérôme**
  **38640 Claix (FR)**

(74) Mandataire: **Lavoix**
**2, place d'Estienne d'Orves**
**75441 Paris Cedex 09 (FR)**

(56) Documents cités:
**EP-A1- 2 182 343       EP-A1- 2 302 362**
**WO-A1-2005/043138     WO-A2-2009/046985**
**US-A1- 2009 266 999**

• **STEINVALL ET AL: "High resolution ladar using time-correlated single-photon counting", SPIE, PO BOX 10 BELLINGHAM WA 98227-0010 USA, vol. 6950, 1 janvier 2008 (2008-01-01), XP040437006,**

**Description**

La présente invention concerne un système de reconstruction des propriétés optiques d'un milieu diffusant, le système de reconstruction comprenant entre autres

- au moins une source de rayonnement pulsée propre à éclairer le milieu diffusant,
- au moins un premier détecteur d'un premier type, propre à recevoir un signal émis par le milieu, le ou chaque premier détecteur étant un détecteur résolu en temps,
- des moyens d'élaboration, pour au moins un couple source - premier détecteur, d'une distribution temporelle du signal reçu par le premier détecteur correspondant.

L'invention concerne également un procédé de reconstruction des propriétés optiques d'un milieu diffusant à l'aide d'un tel système de reconstruction.

L'invention concerne notamment le domaine de l'imagerie de diffusion optique, par exemple l'imagerie de fluorescence sur des tissus biologiques, ou l'imagerie des propriétés endogènes de ces tissus (absorption, diffusion), et en particulier l'imagerie moléculaire optique sur l'animal et l'imagerie moléculaire optique sur un organe de l'homme, tel que le cerveau, un sein, le tube digestif ou d'autres organes dans lesquels des fluorophores sont susceptibles d'être injectés.

L'invention s'applique à la détection du cancer du sein, de la prostate ou encore du tube digestif, particulièrement à la détection de pathologies à la surface du tube digestif. L'invention s'applique également à la dermatologie, notamment à la caractérisation d'une réaction d'inflammation cutanée.

On connaît du document EP 2 302 362 A1 un système de reconstruction du type précité. Il comprend une pluralité de sources de rayonnement pulsées pour éclairer le milieu dont on souhaite acquérir une image puis la traiter, et une pluralité de détecteurs recevant les signaux émis par le milieu éclairé. Chaque source pulsée est une source laser impulsionnelle ou une fibre optique reliée à un laser impulsionnel déporté.

Chaque détecteur est un détecteur résolu en temps, c'est-à-dire un détecteur permettant de mesurer la distribution temporelle du temps d'arrivée des photons, également appelée TCPSC (de l'anglais *Time-Correlated Single Photon Counting*). Selon un premier mode de réalisation, chaque détecteur comporte un photomultiplicateur relié à une fibre optique transmettant le signal depuis le milieu étudié vers le détecteur. Selon un deuxième mode de réalisation, chaque détecteur comporte un ensemble de pixels d'une caméra intensifiée avec une porte temporelle, également appelée caméra intensifiée ultra-rapide de type « gated camera ».

Le système de reconstruction comprend des moyens de réalisation, pour chaque couple source-détecteur, d'une distribution temporelle du signal reçu par le détecteur, et un dispositif de détermination, en fonction des moments d'ordre 0 et d'ordre 1 de la distribution temporelle pour chaque couple source-détecteur, de la localisation de fluorophore(s) agencé(s) dans le milieu.

Toutefois, les détecteurs d'un tel système de reconstruction présentent une instabilité temporelle et/ou en amplitude, ce qui engendre des incertitudes affectant la distribution temporelle. La détermination des propriétés optiques d'un milieu diffusant nécessite de considérer des transformées de telles distributions, en particulier des transformées de Mellin, ces transformées permettant d'établir par exemple des moments d'ordre 0 ou d'ordre 1 utilisés lors du processus de reconstruction. On comprend alors que ces instabilités sont susceptibles d'affecter la précision de la reconstruction.

WO 2005/043138 A1 décrit un système de reconstruction des propriétés optiques d'un milieu diffusant, comprenant une source de rayonnement pulsée propre à éclairer le milieu, un premier détecteur d'un premier type, propre à recevoir un signal émis par le milieu, le premier détecteur étant résolu en temps, des moyens d'élaboration, pour le couple source - premier détecteur, d'une distribution temporelle du signal reçu par le premier détecteur correspondant. Le système comprend en outre un deuxième détecteur d'un deuxième type distinct du premier type, le deuxième détecteur étant un capteur CCD propre à acquérir une image du milieu. Le deuxième détecteur est propre à mesurer une intensité du signal émis par le milieu. WO 2005/043138 A1 ne décrit pas un système comprenant des moyens de calcul d'au moins un paramètre de base en combinant une grandeur caractéristique de la distribution temporelle correspondant à au moins un couple source - premier détecteur et l'intensité mesurée pour au moins un couple source - deuxième détecteur, la reconstruction des propriétés optiques étant effectuée à partir du paramètre de base calculé.

Steinvall et al., "High resolution ladar using time-correlated single-photon counting", SPIE, vol. 6950, janvier 2008, décrit un lidar à haute résolution utilisant une méthode TCSPC pour effectuer une détection du temps d'arrivée d'un photon à partir du temps d'émission de l'impulsion, i.e. pour mesurer le temps de vol du photon. Ce document décrit un détecteur résolu en temps, ainsi qu'un capteur CCD aligné avec le système TCSPC.

WO 2009/046985 A2 décrit un système de reconstruction des propriétés optiques, comprenant une source de lumière comportant un laser propre à éclairer un milieu, un premier détecteur d'un premier type et un deuxième détecteur d'un deuxième type, chaque détecteur étant propre à recevoir un signal émis par le milieu. Le deuxième détecteur comporte un capteur CCD. Ce document ne décrit pas un système comprenant un détecteur résolu en temps.

EP 2 182 343 A1 et EP 2 302 362 A1 décrivent un système de reconstruction des propriétés optiques d'un milieu diffusant, comprenant une source de rayonnement pulsée propre à éclairer le milieu diffusant, un détecteur résolu en temps propre à recevoir un signal émis par le milieu et des moyens d'élaboration, pour chaque couple source - détecteur, d'une distribution temporelle du signal reçu par le premier détecteur correspondant. Ces documents ne décrivent pas un système comprenant en outre au moins un deuxième détecteur d'un type distinct de celui du premier détecteur et formé d'un ensemble de pixel(s) d'un capteur d'image propre à acquérir une image du milieu.

Le but de l'invention est donc de proposer un système de reconstruction des propriétés optiques d'un milieu diffusant, permettant de réduire l'influence des fluctuations parasites des distributions temporelles obtenues par les détecteurs optiques résolus en temps, afin d'améliorer la reconstruction des propriétés optiques.

A cet effet, l'invention, définie selon la revendication 1, a pour objet un système de reconstruction du type précité, comprenant en outre au moins un deuxième détecteur d'un deuxième type distinct du premier type, le ou chaque deuxième détecteur étant formé d'un ensemble de pixel(s) d'un capteur d'image propre à acquérir une image du milieu. Le ou chaque deuxième détecteur est propre à mesurer une intensité du signal émis par le milieu, ladite intensité correspondant à un moment d'ordre 0 équivalent pour le couple source - deuxième détecteur correspondant. Le système est caractérisé en ce qu'il comprend en outre des moyens de calcul d'au moins un paramètre de base en combinant une grandeur caractéristique de la distribution temporelle correspondant à au moins un couple source - premier détecteur et l'intensité mesurée pour au moins un couple source - deuxième détecteur, la reconstruction des propriétés optiques étant effectuée à partir du paramètre de base calculé.

Suivant d'autres aspects avantageux de l'invention, le système de reconstruction comprend une ou plusieurs des caractéristiques suivantes, prises isolément ou suivant toutes les combinaisons techniquement possibles :

- le ou chaque deuxième détecteur est un détecteur non résolu en temps,
- le système comprend des moyens de calcul du moment d'ordre 0 de la distribution temporelle pour chaque couple source - premier détecteur et du moment d'ordre 0 équivalent pour chaque couple source - deuxième détecteur,
- le système comprend des moyens de calcul du seul moment d'ordre 1 de la distribution temporelle pour chaque couple source - premier détecteur,
- le système comprend un dispositif de détermination, en fonction des moments d'ordre 0 et d'ordre 1 calculés, de la localisation de N fluorophore(s) dans le milieu, N étant un nombre entier supérieur ou égal à 1, chaque premier détecteur étant propre à recevoir un signal de fluorescence émis par le fluorophore et chaque deuxième détecteur étant propre à mesurer l'intensité du signal de fluorescence émis,
- le dispositif de détermination comporte des moyens de réalisation d'un maillage d'un volume du milieu, le maillage comportant M maille(s) élémentaires, M étant un nombre entier supérieur ou égal à 1, et des moyens de répartition des N fluorophore(s) dans le milieu, chaque fluorophore étant réparti dans une des M maille(s) du milieu selon une configuration, et des moyens de calcul d'au moins un paramètre de base combinant, pour au moins une distribution temporelle, au moins une grandeur obtenue à partir d'au moins un moment de ladite distribution et au moins une estimation de cette grandeur,
- le système comprend en outre une lentille de focalisation des signaux émis par le milieu, chaque deuxième détecteur étant disposé dans le plan image de la lentille,
- le système comprend en outre un filtre optique agencé entre le milieu et le capteur d'image, de manière à filtrer les signaux émis par le milieu à destination du capteur d'image,
- chaque premier détecteur comporte une fibre optique couplée à un module de détection résolu en temps,
- le capteur d'image est un capteur CCD ou un capteur CMOS,
- les propriétés optiques comprennent au moins un élément parmi le groupe consistant en :

  + les propriétés d'absorption de la lumière, caractérisées notamment par le coefficient d'absorption,
  + les propriétés de diffusion, caractérisées notamment par le coefficient de diffusion réduit ou par le coefficient de diffusion, et
  + les propriétés de fluorescence, caractérisées notamment par une fonction de réponse d'un fluorophore, ou par une concentration du fluorophore, ou encore par une grandeur fonction d'une quantité du fluorophore,

- le système comprend des moyens de calcul d'un facteur de correction associé à la distribution temporelle d'au moins un couple source - premier détecteur, le facteur de correction étant fonction de l'intensité mesurée par un deuxième détecteur correspondant et du moment d'ordre 0 calculé pour ledit au moins un couple source - premier détecteur.

L'invention a également pour objet un procédé de reconstruction de propriétés optiques d'un milieu diffusant, défini selon la revendication 13.

Suivant un autre aspect avantageux de l'invention, le procédé de reconstruction comprend en outre le calcul du moment d'ordre 0 de la distribution temporelle pour chaque couple source - premier détecteur et du moment d'ordre 0 équivalent pour chaque couple source - deuxième détecteur.

Ces caractéristiques et avantages de l'invention apparaîtront à la lecture de la description qui va suivre, donnée uniquement à titre d'exemple, et faite en référence aux dessins annexés sur lesquels :

- la figure 1 est une représentation schématique d'un système de reconstruction selon l'invention, comprenant une sonde d'imagerie comportant des sources de rayonnement pulsées et des premiers et deuxièmes détecteurs ;
- la figure 2 est une vue de face de la sonde de la figure 1 ;
- la figure 3 est une coupe selon le plan III de la figure 2 ; et

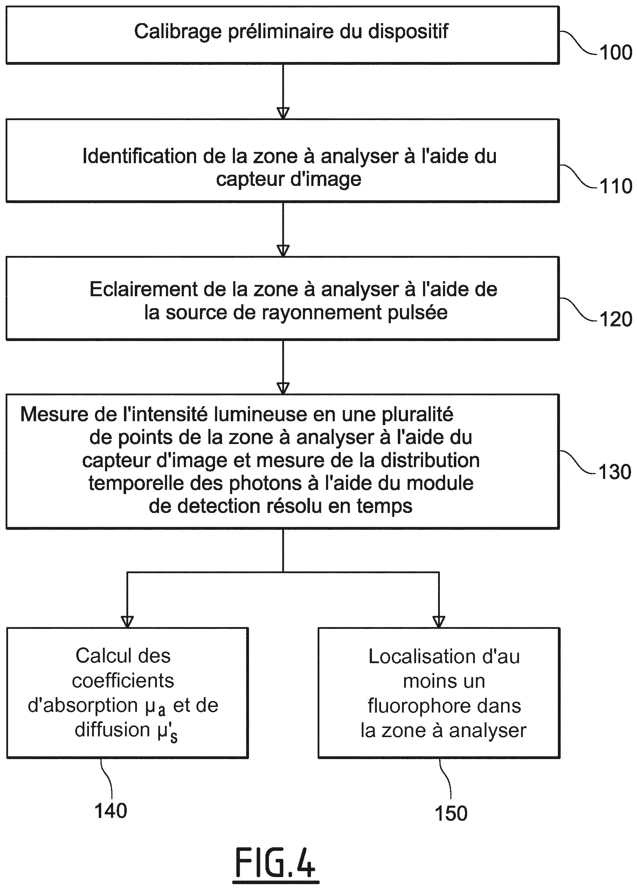

- la figure 4 est un organigramme d'un procédé de reconstruction selon l'invention.

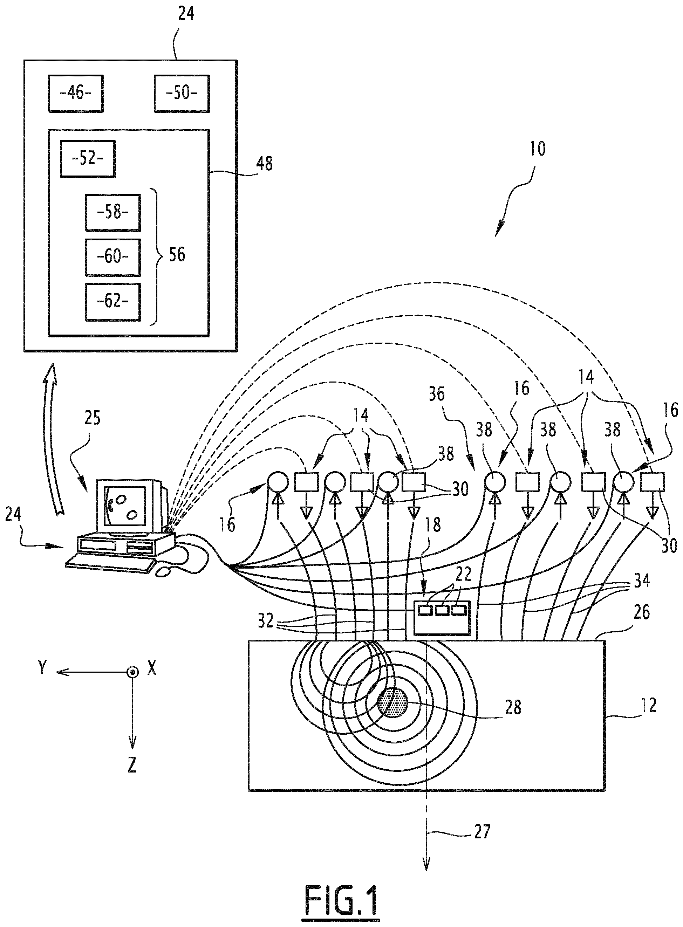

Sur la figure 1, un système de reconstruction 10 est destiné à réaliser un examen d'un milieu diffusant 12, tel un tissu biologique, via l'acquisition de signaux optiques émis par milieu 12 sous l'effet d'un rayonnement d'excitation, puis le traitement de ces signaux pour reconstruire les propriétés optiques de ce milieu.

Par reconstruction des propriétés optiques, on entend, par exemple :

- la reconstruction des propriétés d'absorption, ces dernières étant notamment caractérisées par le coefficient d'absorption, noté $\mu_a$, ou
- la reconstruction des propriétés de diffusion, ces dernières étant notamment caractérisées par le coefficient de diffusion réduit $\mu'_s$ ou par le coefficient de diffusion D', ou encore,
- la reconstruction des propriétés de fluorescence, ces dernières étant notamment caractérisées par une fonction de réponse (F) d'un fluorophore, ou par une concentration c d'un fluorophore, ou encore par toute autre grandeur exprimant une quantité q d'un fluorophore, ce dernier étant par exemple endogène ou exogène.

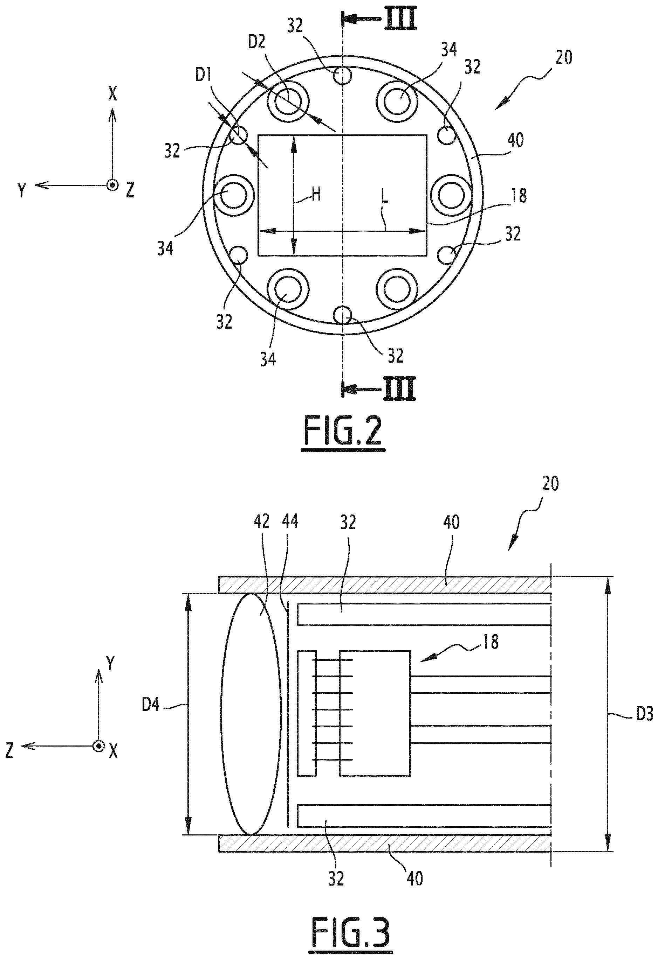

Le système de reconstruction 10 comprend une pluralité de sources de rayonnement pulsées 14 aptes à éclairer le milieu 12, une pluralité de premiers détecteurs 16 d'un premier type, propres à recevoir un signal émis par le milieu 12, et un capteur d'image 18 apte à acquérir une image du milieu 12. Les sources de rayonnement 14, les premiers détecteurs 16 et le capteur d'image 18 sont agencés dans une sonde 20, visible sur les figures 2 et 3.

Le système de reconstruction 10 comprend une pluralité de deuxièmes détecteurs 22 d'un deuxième type distinct du premier type, et chaque deuxième détecteur 22 est formé d'un pixel ou d'un ensemble de pixels du capteur d'images 18.

Le système 10 comprend une unité de traitement d'information 24 et un écran 25 d'affichage d'une image du milieu 12.

En complément, le système 10 comprend, de façon optionnelle, une source de lumière blanche, non représentée, pour fournir un éclairage d'appoint du milieu 12.

Le milieu 12 présente une surface d'observation 26, en forme d'un plan parallèle à un axe longitudinal X et un axe transversal Y, et au contact de laquelle la sonde 20 est propre à être appliquée. Le milieu 12 présente une direction d'observation 27 s'étendant selon un axe vertical Z et sensiblement perpendiculaire à la surface d'observation 26. La profondeur du milieu 12 est mesurée à partir de la surface d'observation 26 et dans le sens de la direction d'observation 27.

Le milieu 12 comporte des tissus biologiques d'un animal ou de l'homme. Le milieu 12 est, par exemple, une zone d'un organe tel que le cerveau, un sein, la prostate, le tube digestif ou encore d'autres organes dans lesquels des fluorophores sont susceptibles d'être injectés.

Dans l'exemple de réalisation de la figure 1, le milieu 12 est un milieu diffusant contenant des inclusions fluorescentes 28, également appelées fluorophores. Un seul fluorophore 28 est représenté sur la figure 1 pour la clarté du dessin.

Chaque source de rayonnement 14 comporte une source lumineuse pulsée 30, également représentée par un indice s, et une fibre optique d'excitation 32 reliée à la source pulsée 30 pour la transmission de l'impulsion lumineuse jusqu'au milieu 12.

En variante non représentée, chaque source de rayonnement 14 comporte une fibre optique d'excitation 32 reliée à une unique source lumineuse pulsée commune à la pluralité de sources de rayonnement 14. Selon cette variante, le système 10 comprend en outre un commutateur optique ou un multiplexeur pour sélectionner la fibre d'excitation 32 dans laquelle le faisceau lumineux est envoyé. On parle alors de source fibrée.

D'une façon générale, lorsque la source lumineuse est couplée à une fibre optique d'excitation 32, l'extrémité de la fibre optique d'excitation assimilée à la source s.

En variante encore, l'ensemble des sources de rayonnement 14 est formé d'une unique source lumineuse pulsée et d'un dispositif à miroir de type MEMS (de l'anglais *MicroElectroMechanical Systems*), non représentés, pour balayer le milieu 12 avec la lumière issue de la source lumineuse puisée.

La longueur d'onde de chaque source de rayonnement 14 est, de préférence, dans le domaine du rouge ou du proche infrarouge, c'est-à-dire comprise entre 500 nm et 1300 nm. Le taux de répétition des impulsions de la source est d'environ 50 MHz.

Les impulsions émises par chaque source de rayonnement 14 présentent une durée comprise entre 500 picosecondes et 50 femtosecondes, chaque source lumineuse pulsée 30 étant propre à générer une impulsion de largeur temporelle de durée comprise entre 500 picosecondes et 50 femtosecondes.

Chaque premier détecteur 16 est un détecteur résolu en temps. Chaque premier détecteur 16 comporte une fibre optique de détection 34 reliée à un module de détection résolu en temps 36. Par détecteur résolu en temps, on entend un détecteur apte à établir une distribution temporelle du signal optique de diffusion émis par le milieu en réponse à une excitation lumineuse impulsionnelle.

D'une façon générale, lorsque le détecteur est couplé à une fibre optique de détection 34, l'extrémité de la fibre optique de détection est assimilée à la source d.

Dans l'exemple de réalisation de la figure 1, le module de détection 36 comporte un organe de détection 38 pour chaque premier détecteur 16. En variante non représentée, le module de détection 36 comporte un organe de détection commun à plusieurs premiers détecteurs 16, en particulier un unique organe de détection pour l'ensemble des premiers détecteurs 16.

Le premier type du premier détecteur 16 est fonction notamment de l'organe de détection 38, l'organe de détection 38 étant, par exemple, un photomultiplicateur, ou une photodiode à avalanche, également appelée APD (de l'anglais *Avalanche PhotoDiode*), ou une photodiode à avalanche à photon unique, également appelée SPAD (de l'anglais *Single-Photon Avalanche Diode*), ou encore un tube intensificateur d'images ayant une ou plusieurs galettes de microcanaux (de l'anglais *Micro-Channel Plate*).

Le capteur 18 est un capteur d'image en deux dimensions, à savoir dans un plan parallèle aux axes X et Y.

Le capteur 18 est, de préférence, un capteur d'image pixellisé, tel un CCD (de l'anglais *Charge-Coupled Device*). En variante, le capteur 18 est un capteur CMOS (de l'anglais *Complementary Metal-Oxide Semiconductor*).

Le capteur d'image 18 présente une résolution haute définition, c'est-à-dire avec un minimum de 720 pixels en hauteur, selon l'axe X sur la figure 2. Le capteur d'images 18 présente selon l'axe X une hauteur H d'environ 1,5 mm et selon l'axe Y une largeur L d'environ 2 mm.

La sonde 20 comporte un boîtier de protection 40 dans lequel sont agencés les fibres d'excitation 32 et de détection 34 et le capteur d'image 18, comme représenté sur les figures 2 et 3. Dans l'exemple de réalisation de la figure 2, le capteur d'images 18 est disposé sensiblement au centre des fibres optiques 32, 34, et les fibres d'excitation 32 et de détection 34 sont agencées en alternance et de manière circulaire à la périphérie du capteur d'image 18.

La sonde 20 comporte une lentille 42 de focalisation des signaux émis par le milieu, comme représenté sur la figure 3. Chaque deuxième détecteur 22 est disposé dans le plan image de la lentille 42. Chaque deuxième détecteur 16 est également susceptible d'être disposé dans le plan image de la lentille 42.

En complément, dans le cas d'une imagerie de fluorescence, la sonde 20 comprend un filtre optique 44 agencé entre le milieu 12 et le capteur d'images 18, de manière à filtrer les signaux émis par le milieu 12 à destination des premiers détecteurs 16 et des deuxièmes détecteurs 22 Dans l'exemple de réalisation de la figure 3, le filtre optique 44 est placé entre la lentille de focalisation 42 et le capteur d'image 18. Ce filtre a pour fonction d'éliminer les signaux optiques dont la longueur d'onde est différente d'une longueur d'onde d'intérêt. Lorsqu'on souhaite reconstruire la répartition des fluorophores dans un milieu, cette longueur d'onde d'intérêt est la longueur d'onde de fluorescence. Lorsqu'on souhaite reconstruire les propriétés optiques de diffusion ou d'absorption du milieu, la longueur d'onde d'intérêt est la longueur d'onde d'excitation de la source.

La sonde 20 est une sonde compacte pour le diagnostic de certains cancers, telle qu'une sonde portative pour le diagnostic du cancer du sein, une sonde endorectale pour le diagnostic du cancer de la prostate, ou encore une sonde de dermatologie.

En variante, la sonde 20 est une sonde d'endoscope, telle qu'une sonde souple pour le diagnostic du cancer digestif, le système de reconstruction 10 étant particulièrement adapté à la détection de pathologies à la surface du tube digestif.

Chaque deuxième détecteur 22 est propre à mesurer une intensité $I_{sd'}$ du signal émis par le milieu 12 lorsque le milieu est éclairé par une source de lumière 14, s et d' représentant respectivement un indice de la source 14 et un indice du deuxième détecteur 22. L'intensité $I_{sd'}$ mesurée est fonction du niveau de gris du ou des points de l'image acquise par le capteur 18 associés au(x) pixel(s) du capteur formant le deuxième détecteur 22 correspondant, l'indice sd' désignant le couple source- deuxième détecteur mis en oeuvre.

Le deuxième type du deuxième détecteur 22 est distinct du premier type du premier détecteur 16, chaque deuxième détecteur 22 étant formé d'un ensemble de pixels du capteur d'images 18, alors que dans le mode de réalisation décrit, chaque premier détecteur 16 comporte une fibre optique de détection 34 reliée à un module de détection résolu en temps 36.

Dans le mode de réalisation décrit, chaque deuxième détecteur 22 est un détecteur non résolu en temps.

L'unité de traitement d'information 24, visible sur la figure 1, comporte un processeur de données 46 et une mémoire 48 associée au processeur 46.

L'unité de traitement 24 comporte des moyens 50 d'élaboration, pour au moins un couple source 14 - premier détecteur 16, d'une distribution temporelle notée M$_{sd}$(t) du signal reçu par le premier détecteur 16 correspondant, s et d représentant respectivement l'indice de la source 14 et un indice du premier détecteur 16. Les moyens d'élaboration 50 sont, de préférence, réalisés sous forme d'une ou plusieurs cartes électroniques connectées aux premiers détecteurs 14 et permettant de mesurer la distribution temporelle du temps d'arrivée des photons, également appelée TCPSC (de l'anglais *Time-Correlated Single Photon Counting*).

Chaque source lumineuse pulsée 30 comporte un laser impulsionnel. En variante, chaque source lumineuse pulsée 30 comporte une diode laser. En variante encore, chaque source lumineuse pulsée 30 comporte une source de lumière constante dont l'intensité lumineuse est modulée en des impulsions de durées équivalentes par un dispositif à obturation rapide. Le dispositif à obturation rapide est un actuateur piézoélectrique, un cristal acousto-optique, une cellule de Pockels ou encore une cellule électro-optique.

Dans l'exemple de réalisation des figures 1 et 3, l'axe optique de chaque fibre optique d'excitation 32 s'étend, au voisinage de l'extrémité de la fibre, selon l'axe Z et perpendiculairement à la surface d'observation 26. Les extrémités des fibres d'excitation 32 sont distribuées selon un plan perpendiculaire à l'axe Z et parallèle à la surface d'observation 26. En variante non représentée, l'axe optique de chaque fibre optique d'excitation 32 s'étend, au voisinage de l'extrémité de la fibre, de manière oblique par rapport à l'axe Z, afin d'émettre la lumière en biais par rapport à la surface d'observation 26, en interceptant, de préférence, le champ du capteur d'image 18. Chaque fibre optique d'excitation 32 présente un premier diamètre D1, de valeur sensiblement constante d'une fibre 32 à l'autre, comme représenté sur la figure 2. La valeur du premier diamètre D1 est, de préférence égale à 65 $\mu$m, et typiquement comprise entre 10 $\mu$m et 1 mm.

Chaque fibre optique de détection 34 présente un deuxième diamètre D2, de valeur sensiblement constante d'une fibre 34 à l'autre. La valeur du deuxième diamètre D2 est, de préférence, égale à 0,5 mm, et typiquement comprise entre 10 $\mu$m et 1 mm voire 5 mm. La valeur du deuxième diamètre D2 est généralement supérieure à celle du premier diamètre D1. Ceci permet de collecter davantage de signal de fluorescence.

Le boitier de protection 40 présente dans un plan parallèle aux axes X et Y un troisième diamètre D3 de valeur, de préférence, comprise entre 1,5 mm et 3,5 mm, comme représenté sur la figure 3.

La lentille de focalisation 42 présente dans un plan parallèle aux axes X et Y un quatrième diamètre D4 de valeur, de préférence, comprise entre 1 mm et 3 mm. La lentille de focalisation 42 offre un champ de vision large, de préférence compris entre 45° et 90°, et une grande profondeur de champ, de préférence comprise entre 1 mm et 40 mm.

La lentille 42 est une lentille à focale fixe. En variante, la lentille 42 est une lentille à focale variable, pour faire le point automatiquement ou pour changer la taille du champ couvert.

Le filtre optique 44 est un filtre de type passe-bande avec une bande passante centrée sur la longueur d'onde d'intérêt.

Le filtre optique 44 est en forme d'un film disposé entre la lentille 42 et le capteur d'image 18. En variante non représentée, le filtre optique 44 est constitué d'un dépôt en couche mince sur le capteur d'image 18.

La mémoire 48 est apte à stocker un premier logiciel 52 de calcul d'un paramètre lié à la distribution temporelle obtenue par chaque premier détecteur 16 lorsque le milieu est excité par une source impulsionnelle 14. Chaque distribution temporelle est notée M$_{sd}$(t), l'indice sd désignant le couple source- premier détecteur considéré. Le premier logiciel établit alors une grandeur caractéristique de cette distribution temporelle M$_{sd}$(t). Cette grandeur est notamment obtenue par une transformée de la distribution temporelle M$_{sd}$(t) considérée. La transformée est, par exemple, une transformée de Mellin, permettant de déterminer le moment d'ordre 0, noté M$^{(0)}_{sd}$, de la distribution temporelle M$_{sd}$(t) pour chaque couple source 14 - premier détecteur 16. Une telle transformée permet également de déterminer un moment d'ordre 1 noté M$^{(1)}_{sd}$, de la distribution temporelle (M$_{sd}$(t)) pour chaque couple source 14 - premier détecteur 16.

Le moment d'ordre 0 M$^{(0)}_{sd}$ d'une distribution temporelle correspond à l'intensité I$_{sd}$ du signal émis par le milieu, c'est-à-dire à la quantité de photons détectés. Comme précédemment évoqué, le deuxième détecteur 22 n'est pas résolu en temps, et il n'est donc pas apte à déterminer la distribution temporelle du signal optique détecté en réponse à une impulsion d'une source d'excitation 14. Par contre, ce deuxième détecteur 22 mesure directement une intensité I$_{sd'}$ du signal optique émis par le milieu en réponse à une excitation. Une telle intensité I$_{sd'}$ est assimilable à un moment d'ordre M$^{(0)}_{sd'}$ du signal optique émis par le milieu en réponse à l'excitation par la source 14. Autrement dit, la mesure I$_{sd'}$ du deuxième détecteur est équivalente à un moment d'ordre 0. En d'autres termes, la mesure I$_{sd'}$ effectuée par le deuxième détecteur correspond à un moment d'ordre 0 équivalent M$^{(0)}_{sd}$ pour le couple source 14- deuxième détecteur 22 associé l'indice sd'.

Les moments M$^{(0)}_{sd}$, M$^{(0)}_{sd'}$ d'ordre 0 vérifient les équations suivantes :

$$M_{sd}^{(0)} = \int_{0}^{\infty} M_{sd}(t).t^{0}dt = I_{sd} \qquad\qquad (1)$$

Le moment $M^{(1)}_{sd}$ d'ordre 1 vérifie l'équation suivante :

$$M^{(1)}_{sd} = \int_0^\infty M_{sd}(t).t^1 dt \qquad (2)$$

D'une façon générale, le moment $M^{(n)}_{sd}$ d'ordre n vérifie l'équation suivante :

$$M^{(n)}_{sd} = \int_0^\infty M_{sd}(t).t^n dt \qquad (3)$$

En variante, les premiers moyens de calcul 52 sont réalisés sous forme de composants logiques programmables ou encore sous forme de circuits intégrés dédiés.

L'unité de traitement d'information 24 comporte un dispositif 56 de détermination, en fonction des moments d'ordre 0 et d'ordre 1 calculés $M^{(0)}_{sd}$, $M^{(0)}_{sd}$, $M^{(1)}_{sd}$, de la localisation de N fluorophore(s) 28 dans le milieu 12, avec $M^{(0)}_{sd}$ , $= I_{sd'}$, chaque premier détecteur 14 étant alors propre à recevoir un signal de fluorescence émis par le fluorophore 28 et chaque deuxième détecteur 22 étant propre à mesurer l'intensité $I_{sd'}$ du signal de fluorescence émis. N est un nombre entier supérieur ou égal à 1.

Le dispositif de détermination 56 comporte un logiciel 58 de réalisation d'un maillage d'un volume du milieu 12, le maillage comportant M maille(s) élémentaires, M étant un nombre entier supérieur ou égal à 1, et un logiciel 60 de répartition des N fluorophore(s) 28 dans le milieu 12, chaque fluorophore 28 étant réparti dans une des M maille(s) du milieu selon une configuration m.

Le dispositif de détermination 56 comporte un deuxième logiciel 62 de calcul d'au moins un paramètre de base $X^N_{j,m}$ combinant, pour au moins une distribution temporelle $M_{sd}(t)$ détectée par un premier détecteur 16, au moins une grandeur obtenue à partir d'au moins un moment de ladite distribution et au moins une estimation de cette grandeur, j représentant un indice du paramètre de base calculé. De même, le paramètre de base $X^N_{j,m}$ combine une grandeur $M^{(0)}_{sd}$ issue du signal mesuré par un deuxième détecteur 22 et une estimation $M^{theo0}_{sd'}$ de ladite grandeur

Le logiciel de réalisation d'un maillage 58, le logiciel de répartition des fluorophores 60 et le deuxième logiciel de calcul 62 sont propres à être stockés dans la mémoire 48.

La réalisation du maillage, la répartition des fluorophores 28 et le calcul des paramètres de base $X^N_{j,m}$ sont expliqués dans le document EP 2 302 362 A1 publié le 30 mars 2011, en particulier dans les passages de la page 6, ligne 25 à la page 11, ligne 45 et de la page 14, ligne 24 à la page 25, ligne 6, en regard des figures associées à ces passages, avec les paramètres de base $X^N_{1,m}$, $X^N_{2,m}$, $X^N_{4,m}$, vérifiant les équations suivantes pour la présente invention :

$$\chi^N_{1,m} = \min_{\alpha_1,\alpha_2,...,\alpha_N} \left[ \sum_{sd} \frac{\left(M^{(0)}_{sd} - M^{theo0}_{sd,m}(\alpha_1,\alpha_2,...,\alpha_N)\right)^2}{\sigma^2\left(M^{(0)}_{sd}\right)} + \sum_{sd'} \frac{\left(M^{(0)}_{sd'} - M^{theo0}_{sd',m}(\alpha_1,\alpha_2,...,\alpha_N)\right)^2}{\sigma^2\left(M^{(0)}_{sd'}\right)} \right] \quad (4)$$

$$\chi^N_{2,m} = \min_{\alpha_1,\alpha_2,...,\alpha_N} \sum_{sd} \frac{\left((T_{sd} - T_s - \tau - T_d) - (T^{theo0}_{sd,m}(\alpha_1,\alpha_2,...,\alpha_N))\right)^2}{\sigma^2\left(T_{sd}\right)} \quad (5)$$

$$\chi^N_{4,m} = \min_{\alpha_1,\alpha_2,...,\alpha_N} \left[ \sum_{sd} \frac{\left(M^{(0)}_{sd} - M^{theo0}_{sd,m}(\alpha_1,\alpha_2,...,\alpha_N)\right)^2}{\sigma^2\left(M^{(0)}_{sd}\right)} + \sum_{sd'} \frac{\left(M^{(0)}_{sd'} - M^{theo0}_{sd',m}(\alpha_1,\alpha_2,...,\alpha_N)\right)^2}{\sigma^2\left(M^{(0)}_{sd'}\right)} + \sum_{sd} \frac{\left((T_{sd} - T_s - \tau - T_d) - (T^{theo0}_{sd,m}(\alpha_1,\alpha_2,...,\alpha_N))\right)^2}{\sigma^2\left(T_{sd}\right)} \right] \quad (6)$$

où $T_{sd}$ représente le temps de vol entre la source 14 et le premier détecteur 16,
$T_g$ représente le temps de réponse moyen de la source 14,
$\tau$ représente la durée de fluorescence du fluorophore 28,

$T_d$ représente le temps de réponse moyen du premier détecteur 16,

$\sigma(X)$ représente une grandeur statistique relative à l'estimation de la grandeur X. Ce terme de normalisation est facultatif. Il traduit notamment la précision de cette estimation. Il s'agit, par exemple, d'un écart-type ou d'une variance.

$\alpha_1, \alpha_2, ..., \alpha_N$, sont des coefficients affectés chacun à un fluorophore 28 correspondant et représentant l'intensité d'émission de la fluorescence par celui-ci. Ces coefficients sont obtenus lors de l'opération de minimisation correspondant aux équations (4), (5) ou (6).

Le temps de vol $T_{sd}$ vérifie l'équation suivante :

$$T_{sd} = \frac{\int_0^{\infty} M_{sd}(t).t^1.dt}{\int_0^{\infty} M_{sd}(t).t^0.dt} = M_{sd}^{(0)} / M_{sd}^{(1)} \tag{7}$$

Autrement dit, les termes des paramètres de base $X^N_{1,m}$ et $X^N_{4,m}$ comportant des moments d'ordre 0 sont calculés avec les moments $M^{(0)}_{sd}$, $M^{(0)}_{sd}$ pour les premiers et deuxièmes détecteurs 16, 22, alors que les termes des paramètres de base $_X N^2_{2,m}$ et $X^N_{4,m}$ comportant des moments d'ordre 1 sont calculés avec les moments $M^{(1)}_{sd}$ pour les premiers détecteurs 16 seulement.

Ainsi, d'une façon générale, on combine une grandeur caractéristique de la distribution temporelle $M_{sd}(t)$ correspondant à au moins un couple source 14 - premier détecteur 16 (indice sd) et l'intensité $I_{sd'}$ mesurée pour au moins un couple source 14-deuxième détecteur 22 (indice sd'). La combinaison donne alors lieu à un paramètre de base $X^N_{1,m}$, $X^N_{2,m}$, $X^N_{4,m}$, à partir duquel la reconstruction de la fluorescence est réalisée.

Il est utile d'utiliser le moment d'ordre 1 associé à un couple source- premier détecteur 16. En effet, les inventeurs ont montré que l'utilisation d'un tel moment permet d'obtenir le temps de vol $T_{sd}$, cette grandeur étant particulièrement représentative de la profondeur du fluorophore dans le milieu. D'autre part, le moment d'ordre 0 est plus sensible à la position du fluorophore dans un plan parallèle à la surface du milieu, c'est-à-dire dans le plan d'axes X et Y dans l'exemple de réalisation de la figure 1.

On comprend alors qu'en utilisant des paramètres de base $X^N_{1,m}$, $X^N_{2,m}$, $X^N_{4,m}$, combinant des moments d'ordre 1 et des moments d'ordre 0, on obtient une information précise sur la position du détecteur dans le plan d'axes X et Y, mais également sur la profondeur selon l'axe Z.

Comme précédemment évoqué, la distribution temporelle $M_{sd}(t)$ associée à un couple source-détecteur est susceptible de fluctuer du fait de l'instabilité d'un premier détecteur 16 résolu en temps. On comprend alors que les paramètres de base définis ci-dessus seront d'autant plus robustes qu'ils sont établis à partir des intensités $I_{sd'}(t)$, équivalents à des moments d'ordre 0 $M^{(0)}_{sd'}$, mesurés sur des détecteurs dont la réponse est stable, c'est-à-dire des détecteurs 22 du deuxième type, non résolus en temps.

Un avantage de l'invention est alors de combiner :

- des mesures d'au moins un premier détecteur 16 résolu en temps, car elles permettent de déterminer le temps de vol $T_{sd}$, et, partant de là, une information sur la profondeur du fluorophore,
- des intensités $I_{sd'}$ mesurées par au moins un deuxième détecteur 22, non résolu en temps, ces dernières étant plus stables, et porteuses d'information sur la position du fluorophore dans un plan d'axes X et Y, perpendiculaire à l'axe Z définissant la profondeur.

On notera également que lorsque les termes $\sum_{sd} \frac{\left(M_{sd}^{(0)} - M_{sd,m}^{theo0}(\alpha_1, \alpha_2, ..., \alpha_N)\right)^2}{\sigma^2\left(M_{sd}^{(0)}\right)}$ et $\sum_{sd'} \frac{\left(M_{sd'}^{(0)} - M_{sd',m}^{theo0}(\alpha_1, \alpha_2, ..., \alpha_N)\right)^2}{\sigma^2\left(M_{sd'}^{(0)}\right)}$ sont combinés, le coefficient de normalisation $\sigma^2\left(M_{sd}^{(0)}\right)$ sera, par exemple, inférieur au coefficient de normalisation $\sigma^2\left(M_{sd'}^{(0)}\right).$ Cela permet de pondérer l'effet de mesures obtenues à l'aide de détecteurs instables, au profit de mesures obtenues à l'aide de détecteurs stables. On rappelle que l'utilisation de tels coefficients de normalisation est optionnelle.

En variante, les moyens de réalisation d'un maillage 58, les moyens de répartition des fluorophores 60 et les deuxièmes moyens de calcul 62 sont réalisés sous forme de composants logiques programmables ou encore sous forme de circuits intégrés dédiés.

Le fonctionnement du système de reconstruction 10 selon l'invention est décrit ci-après à l'aide de la figure 3 représentant un organigramme du procédé de reconstruction selon l'invention.

Préalablement à son utilisation, le système 10 est calibré, lors de l'étape 100, à l'aide d'un fantôme ayant des caractéristiques optiques connues. Le fantôme est un fantôme ayant des coefficients d'absorption $\mu_a$, et de diffusion $\mu_s'$ de valeurs médianes pour les tissus humains, par exemple égales à 0,2 cm$^{-1}$, et respectivement à 10 cm$^{-1}$. Ce calibrage préliminaire 100 permet de vérifier le fonctionnement du système 10, et de le paramétrer en fonction d'éventuels biais détectés. Ces éventuels biais sont, par exemple, dus à une variation de la puissance des sources 14, à la présence de poussières sur la lentille 42, et/ou à la modification de l'efficacité de couplage des fibres optiques 32, 34 en raison de variations de température.

Une zone à analyser du milieu 12 est ensuite identifiée, lors de l'étape 110, à l'aide du capteur d'image 18. Les tissus du milieu 12 sont éclairés par chaque source de lumière pulsée 30 avec une fréquence telle que le capteur d'image 18 la perçoit comme une lumière continue. Cette fréquence est, de préférence, comprise entre 10 et 100 MHz. Le temps d'exposition du capteur d'image 18, compris entre 1 ms et 100 ms, ne permet alors pas de distinguer les impulsions lumineuses, et l'image affichée à l'écran 25 à l'aide du capteur 18 ne présente pas de clignotement.

Après identification de la zone à analyser, celle-ci est éclairée par chaque source de rayonnement pulsée 14 (étape 120), et la lumière pulsée est alors rétrodiffusée par les tissus du milieu 12. Cette lumière rétrodiffusée est collectée par les deuxièmes détecteurs 22 du capteur d'image, ainsi que par les premiers détecteurs 16 dont les fibres de détection 34 sont placées autour du capteur d'image 18.

Les deuxièmes détecteurs 22 permettent de mesurer directement le moment d'ordre 0 équivalent $M^{(0)}_{sd'}$, en l'absence de l'élaboration d'une distribution temporelle pour les deuxièmes détecteurs 22. Les fibres optiques de détection 34 conduisent la lumière au module de détection résolu en temps 36, et les moyens d'élaboration 50, reliés au module de détection résolu en temps 36, permettent alors d'élaborer la distribution temporelle $M_{sd}(t)$ des temps d'arrivée des photons dans chaque fibre de détection 34 (étape 130).

Le système 10 dispose donc deux types de mesure, à savoir des mesures d'intensité $I_{sd'}$ en un grand nombre de points, directement fournies par le capteur d'image 18, et des mesures de la distribution $M_{sd}(t)$ temporelle des photons captés par les fibres de détection 34, à l'aide du module de détection résolu en temps 36 connecté à ces fibres de détection 34. Ceci permet de déterminer une carte des propriétés optiques (étape 140) et/ou de localiser les N fluorophore(s) 28 dans la zone à analyser (étape 150).

Lors de l'étape 140, la reconstruction vise à déterminer la distribution spatiale des propriétés optiques d'absorption $\mu_a$ et de diffusion D du milieu diffusant 12.

Pour cela, on utilise par exemple la transformée de Mellin-Laplace de la distribution $M_{sd}(t)$ mesurée par un premier détecteur 16.

La transformée de Mellin-Laplace d'ordre n et de largeur p d'une fonction f est notée $f^{(p,n)}$, et vérifie l'équation suivante :

$$f^{(p,n)} = \frac{1}{n!} \int_{0}^{+\infty} (pt)^n . \exp(-pt) . f(t) . dt \qquad (8)$$

où 1/n! représente un terme de normalisation optionnel.

La transformée de Mellin-Laplace $f^{(p,n)}$ de la fonction f s'écrit également :

$$f^{(p,n)} = \int_{-\infty}^{+\infty} W^{(p,n)} . f(t) . dt \qquad (9)$$

où $W^{(p,n)}$ représente une fenêtre temporelle définie par l'équation suivante :

$$W^{(p,n)} = \frac{H(t).(pt)^n.\exp(-pt)}{n!} \qquad (10)$$

avec H(t) représentant la fonction connue de Heaviside.

La reconstruction s'effectue selon un procédé itératif, en réalisant une distribution $M_{sd}(t)$ pour un ou différents couple(s) source-détecteur.

Cette distribution temporelle M$_{sd}$(t) vérifie l'équation suivante :

$$M_{sd}(t) = IRF_{sd}(t) * G_{sd}(t) \tag{11}$$

où IRF$_{sd}$(t) représente la réponse instrument, c'est-à-dire l'influence de la source s et du détecteur d sur la première distribution élaborée,

\* désigne le produit de convolution, et

G$_{sd}$(t) représente une première fonction de modélisation d'un signal de diffusion de la lumière entre la source 14 et le premier détecteur 16 dans le milieu diffusant 12.

On note S$_s$(t) la réponse temporelle de la source 14, également notée s. Cette source 14 est une brève impulsion lumineuse, souvent modélisée par une distribution de type Dirac ponctuelle. S$_s$(t) représente l'intensité temporelle du signal émis.

On note D$_d$(t) la réponse temporelle du premier détecteur 16, également noté d. Cette réponse traduit notamment le délai entre l'arrivée d'un photon sur le détecteur 16 et sa détection effective.

On estime généralement la réponse instrument, notée IRF$_{sd}$(t) l'indice sd corrrespondant à un couple source d'excitation 14 - premier détecteur 16. Cette réponse instrument est obtenue en combinant Ss(t) et Dd(t) suivant : IRF$_{sd}$(t) = S$_s$(t)\*D$_d$(t), où \* désigne le produit de convolution.

Lorsqu'on a plusieurs sources et plusieurs détecteurs, on détermine IRF$_{sd}$(t) pour chaque couple source-détecteur, car chaque source et chaque détecteur a sa propre réponse. En pratique, cela se fait en plaçant une source (ou une extrémité de fibre optique constituant la source) en face d'un premier détecteur (ou d'une extrémité de fibre optique constituant le détecteur). Cela suppose un alignement soigné entre la source et le premier détecteur, et cela prend du temps, notamment lorsqu'on augmente le nombre de sources et de détecteurs. A titre d'exemple, 100 fonctions de réponse instrument IRF$_{sd}$ sont à déterminer lorsqu'on dispose de 10 sources et 10 détecteurs.

Pour chaque couple source-détecteur, on obtient alors l'équation suivante :

$$M_{sd}(t) - M_{sd}^1(t) = -S_s(t) * \left( \int G_s^1(\vec{r},t) * \delta\mu_a(\vec{r},t) * G_d^1(\vec{r},t)d\vec{r} + \int \vec{\nabla}G_s^1(\vec{r},t) * \delta D(\vec{r},t) * \vec{\nabla}G_d^1(\vec{r},t)d\vec{r} \right) * D_d(t) \tag{12}$$

où :

- $M_{sd}^1$ représente une mesure modélisée en considérant que le milieu présente les coefficients d'absorption $\mu_a^1$(r) et de diffusion D$_a^1$(r) déterminés lors de l'itération précédente, les valeurs $\mu_a^1$(r) et D$_a^1$(r) étant initialisées lors de la première itération par une première estimation réalisée par l'opérateur,

- G$_s$(r,t) = G(r$_s$,r,t) est la fonction de Green représentant la densité de photons à un endroit r du milieu 12 lorsque le milieu est éclairé par la source s située en r$_s$,

- G$_d$(r,t) = G(r$_d$,r,t) est la fonction de Green représentant la densité de photons à l'endroit r du milieu 12 lorsque le milieu est éclairé par la source s située en r$_d$. On écrit également G$_d$(r,t) = G(r,r$_d$,t). Ainsi, cette fonction de Green représente également la densité de photons au niveau du détecteur (r$_d$) lorsque le milieu 12 est éclairé par une source située en r.

- les coefficients $\delta\mu_a(\vec{r},t)$ et $\delta D(\vec{r},t)$ représentent la différence entre les propriétés optiques du milieu ($\mu_a$, D) et les propriétés optiques ($\mu_a^1$,D$^1$) initiales, ou bien résultant de l'itération précédente.

L'objectif est de minimiser $\delta\mu_a(\vec{r},t)$ et $\delta D(\vec{r},t)$.

On détermine les transformées de Mellin Laplace d'une fonction Y$_{sd}$, ces transformées se présentant sous la forme :

$$Y_{s,d}^{(p,n)} = \frac{M_{s,d}^{(p,n)} - \sum_{i=0}^{n-1} IRF_{s,d}^{(p,n-i)} M_{s,d}^{(p,i)}}{I_{s,d}^{(p,0)}} \tag{13}$$

Ainsi, en fonction des transformées de Mellin-Laplace de la réponse instrument $IRF_{s,d}^{(p,k)}$, avec $0 \leq k \leq n$, et

de la mesure $M_{s,d}^{(p,k)}$ avec $0 \le k \le n$, on détermine $Y_{s,d}^{(p,n)}$ :

$$Y_{s,d}^{(p,n)} = \left( \int_{\Omega} \sum_{j+k=n} G_s^{1(p,j)}(\vec{r}).\delta\mu_a(\vec{r}).G_d^{1(p,k)}(\vec{r})d\vec{r} + \int_{\Omega} \sum_{j+k=n} G_s^{1(p,j)}(\vec{r}).\delta D(\vec{r}).G_d^{1(p,k)}(\vec{r})d\vec{r} \right) \quad (14)$$

Après discrétisation du milieu en M voxels m, cette expression devient :

$$Y_{s,d}^{(p,n)} = \sum_{m=1}^{M} \left( \sum_{j+k=n} G_s^{1(p,j)}(\vec{r}_m).\delta\mu_a(\vec{r}_m).G_d^{1(p,k)}(\vec{r}_m)V_m + \sum_{j+k=n} G_s^{1(p,j)}(\vec{r}_m).\delta D(\vec{r}_m).G_d^{1(p,k)}(\vec{r}_m)V_m \right) \quad (15)$$

L'étape de reconstruction vise alors à résoudre le système matriciel suivant :

$$\underline{Y} = \underline{W}\,\underline{X}\,, \quad (16)$$

où $\underline{Y}$ représente un vecteur des observations, $\underline{W}$ représente une matrice de passage, et $\underline{X}$ représente un vecteur des inconnues.

Le vecteur des observations Y contient les Nn transformées de Mellin-Laplace de la fonction $Y_{sd}^{(p,n)}$, avec Nn égal à $n_{max}+1$, calculées pour toutes les valeurs successives de l'ordre n comprises entre 0 et $n_{max}$ et pour les couples source-détecteur (s, d) considérés.

On définit, pour chaque triplet (s, d, n) des indices s, d et de l'ordre n, un indice I de la manière suivante :

$$I = (s-1) \times Nd \times Nn + (d-1) \times Nn + n \quad (17)$$

où Nd est le nombre de détecteurs 16 considérés.

La valeur maximale de l'indice I est égale à Imax, tel que :

$$I_{max} = Ns \times Nd \times Nn \quad (18)$$

Le vecteur des observations $\underline{Y}$ comporte alors Imax lignes.

La matrice de passage $\underline{W}$ comprend une première partie $W^\mu$ comportant des premiers termes notés $W^\mu(I,m)$ et une deuxième partie $W^D$ comportant des deuxièmes termes notés $W^D(I,m)$. Cette matrice est appelée matrice de sensibilité des mesures Y aux propriétés optiques X.

La matrice de passage $\underline{W}$ comporte alors Imax lignes et 2M colonnes.

Chaque terme $W^\mu(I,m)$, $W^D(I,m)$ de la matrice de passage $\underline{W}$ est déterminé par modélisation, à chaque itération, en fonction des propriétés optiques déterminées lors de l'itération précédente, ou lors de la première itération, en fonction de propriétés optiques initialisées par l'opérateur. Cette initialisation est réalisée en considérant par exemple un milieu 12 homogène.

Le vecteur des inconnues $\underline{X}$ comporte 2M lignes et une colonne, et contient les inconnues $\mu_a(m)$ et D(m) pour chacun des M voxels. Les M premières lignes correspondent aux inconnues $\mu_a(m)$ et les M lignes suivantes du vecteur $X$ correspondent aux inconnues D(m).

L'inversion de la matrice de passage $\underline{W}$ est réalisée selon des algorithmes d'inversion bien connus de l'homme du métier, tels qu'un algorithme de descente de gradient, une technique de reconstruction algébrique (ART), une décomposition en valeurs singulières (SVD), ou encore une méthode des gradients conjugués. Le processus s'arrête lorsqu'un critère de convergence est atteint, par exemple lorsque la distance entre deux vecteurs $\underline{X}$ successifs est inférieure à un seuil prédéterminé.

Lors de la reconstruction décrite ci-dessus, chaque distribution $M_{sd}(t)$ est susceptible d'être affectée par l'instabilité d'un premier détecteur 16 résolu en temps.

On sélectionne alors un deuxième détecteur 22, dont on mesure l'intensité $I_{sd'}$, et on utilise cette mesure plus stable, pour corriger la distribution temporelle $M_{sd}(t)$. De préférence, le deuxième détecteur 22 est placé à proximité du premier détecteur 16 considéré.

Un exemple de correction est donné par l'équation suivante, M*sd(t) représentant la distribution Msd(t) corrigée, dont les transformées de Mellin Laplace sont déterminées et utilisées pour la reconstruction selon les principes précédemment exposés.

$$M*_{sd}(t) = M_{sd}(t) * \frac{I_{sd'}}{M_{sd}^0} \qquad (19)$$

Ainsi, la combinaison de mesures d'un premier détecteur 16 résolu en temps et d'un deuxième détecteur 22 non résolu en temps permet d'atténuer les effets d'instabilité précédemment décrits. On comprend que le terme de correction $\frac{I_{sd'}}{M_{sd}^0}$ est particulièrement approprié lorsqu'on effectue une série de mesures en utilisant le même couple source-premier détecteur sd et le même couple source-deuxième détecteur sd'.

Ce type de correction n'est pas limité à ce mode de réalisation, et est envisageable lorsqu'on souhaite reconstruire les propriétés de fluorescence du milieu examiné (étape 150).

Lors de l'étape 150, la reconstruction vise à déterminer les propriétés de fluorescence du milieu diffusant. Les N fluorophore(s) 28 sont localisés à l'aide du dispositif de détermination 56 en calculant les paramètres de base $X^N_{1,m}$, $X^N_{2,m}$, $X^N_{4,m}$ $X^N_{5,m}$ à l'aide des équations (4) à (6), étant considéré que les valeurs des paramètres de base $X^N_{1,m}$, $X^N_{2,m}$, $X^N_{4,m}$ $X^N_{5,m}$ sont minimales pour les configurations m les plus proches de la configuration réelle des N fluorophore(s) 28 dans le milieu 12. Le paramètre de base $X^N_{1,m}$ est le paramètre choisi préférentiellement parmi les paramètres de base $X^N_{1,m}$, $X^N_{2,m}$, $X^N_{4,m}$ $X^N_{5,m}$ pour localiser les N fluorophore(s) 28.

Le système de reconstruction 10 selon l'invention calcule donc les moments d'ordre 0 à l'aide de deux types de mesure, à savoir les mesures d'intensité $I_{sd'}$ en un grand nombre de points, celles-ci étant directement fournies par les deuxièmes détecteurs 22 du capteur d'image 18, et les mesures de la distribution $M_{sd}(t)$ temporelle des photons captés par les fibres de détection 34 des premiers détecteurs 16. Ceci permet de réduire les fluctuations parasites des termes des paramètres de base $X^N_{1,m}$ et $X^N_{4,m}$ comportant des moments d'ordre 0, alors que dans le système de reconstruction de l'état de la technique, les moments d'ordre 0 des termes des paramètres de base $X^N_{1,m}$ et $X^N_{4,m}$ sont calculés exclusivement pour la distribution $M_{sd}(t)$ temporelle des photons captés par les détecteurs résolus en temps. Les fluorophores sont ainsi mieux localisés.

En variante, le système de reconstruction 10 calcule les moments d'ordre 0 à l'aide des seules mesures d'intensité $I_{sd'}$ directement fournies par les deuxièmes détecteurs 22 du capteur d'image 18.

Autrement dit, seul le moment d'ordre 1 $M^{(1)}_{sd}$ de la distribution temporelle $M_{sd}(t)$ est calculé pour chaque couple source 14 - premier détecteur 16.

Selon cette variante, les paramètres de base $X^N_{1,m}$, $X^N_{2,m}$, $X^N_{4,m}$ vérifient les équations suivantes :

$$\chi^N_{1,m} = \min_{\alpha_1,\alpha_2,...,\alpha_N} \left[ \sum_{sd'} \frac{\left(M^{(0)}_{sd'} - M^{theo\,0}_{sd',m}(\alpha_1,\alpha_2,...,\alpha_N)\right)^2}{\sigma^2\left(M^{(0)}_{sd'}\right)} \right] \qquad (20)$$

$$\chi^N_{2,m} = \min_{\alpha'_1,\alpha'_2,...,\alpha'_N} \sum_{sd} \frac{\left((T_{sd} - T_s - \tau - T_d) - (T^{theo\,0}_{sd,m}(\alpha_1,\alpha_2,...,\alpha_N))\right)^2}{\sigma^2\left(T_{sd}\right)} \qquad (21)$$

$$\chi^N_{4,m} = \min_{\alpha_1,\alpha_2,...,\alpha_N} \left[ \sum_{sd'} \frac{\left(M^{(0)}_{sd'} - M^{theo\,0}_{sd',m}(\alpha_1,\alpha_2,...,\alpha_N)\right)^2}{\sigma^2\left(M^{(0)}_{sd'}\right)} + \sum_{sd} \frac{\left((T_{sd} - T_s - \tau - T_d) - (T^{theo\,0}_{sd,m}(\alpha_1,\alpha_2,...,\alpha_N))\right)^2}{\sigma^2\left(T_{sd}\right)} \right] \qquad (22)$$

Les termes des paramètres de base $X^N_{1,m}$ et $X^N_{4,m}$ comportant des moments d'ordre 0 ne dépendent alors plus des variations parasites des moments d'ordre 0 calculés à l'aide des premiers détecteurs 16. Les fluorophores sont également mieux localisés.

Selon un autre mode de réalisation, non représenté, les deuxièmes détecteurs 22 ne sont pas situés à proximité des fibres optiques d'excitation 32 et des premiers détecteurs 16, mais sont séparés de ces derniers par le milieu diffusant examiné.

Autrement dit, les premiers détecteurs 16 fonctionnent selon un mode de rétrodiffusion, tandis que les deuxièmes détecteurs 22 fonctionnent selon un mode de transmission. Cette configuration est notamment appropriée à des applications de type mammographie, le sein à analyser étant alors disposé entre la source 14 (ou l'extrémité des fibres d'excitation) et les deuxièmes détecteurs 22. De préférence, les premiers détecteurs 16, résolus en temps, fonctionnent selon un mode de rétrodiffusion, c'est-à-dire qu'ils mesurent un signal optique rétrodiffusé par le milieu, le milieu n'étant pas intercalé entre la source d'excitation 14 et ces premiers détecteurs 16.

Ainsi, les premiers et deuxièmes détecteurs 16, 22 fonctionnent selon un mode de rétrodiffusion, ou bien selon un mode de transmission, l'idée de l'invention étant de combiner leurs signaux respectifs afin de reconstruire les propriétés optiques du milieu diffusant examiné.

On conçoit ainsi que le système de reconstruction 10 selon l'invention permet de réduire l'influence des fluctuations parasites des distributions temporelles obtenues par les détecteurs optiques résolus en temps, afin d'améliorer la reconstruction des propriétés optiques.

**Revendications**

1. Système (10) de reconstruction de propriétés optiques d'un milieu diffusant (12), le système de reconstruction (10) comprenant :

   - au moins une source de rayonnement pulsée (14, s) propre à éclairer le milieu diffusant (12),
   - au moins un premier détecteur (16, d) d'un premier type, propre à recevoir un signal émis par le milieu (12), le ou chaque premier détecteur (16, d) étant un détecteur résolu en temps,
   - des moyens d'élaboration, pour au moins un couple source (14, s) - premier détecteur (16, d), d'une distribution temporelle ($M_{sd}(t)$) du signal reçu par le premier détecteur (16, d) correspondant,
   le système (10) comprenant en outre au moins un deuxième détecteur (22, d') d'un deuxième type distinct du premier type, le ou chaque deuxième détecteur (22, d') étant formé d'un ensemble de pixel(s) d'un capteur d'image (18) propre à acquérir une image du milieu (12), le ou chaque deuxième détecteur (22, d') étant propre à mesurer une intensité ($I_{sd'}$) du signal émis par le milieu (12), ladite intensité ($I_{sd'}$) correspondant à un moment d'ordre 0 équivalent ($M^{(0)}_{sd'}$) pour le couple source (14, s)
   - deuxième détecteur (22, d') correspondant, le système (10) étant **caractérisé en ce qu'**il comprend en outre des moyens (62) de calcul d'au moins un paramètre de base ($X^N_{j,m}$) en combinant une grandeur caractéristique de la distribution temporelle ($M_{sd}(t)$) correspondant à au moins un couple source (14, s) - premier détecteur (16, d) et l'intensité ($I_{sd'}$) mesurée pour au moins un couple source (14, s) - deuxième détecteur (22, d'), la reconstruction des propriétés optiques étant effectuée à partir du paramètre de base ($x^N_{j,m}$) calculé.

2. Système (10) selon la revendication 1, dans lequel le ou chaque deuxième détecteur (22, d') est un détecteur non résolu en temps.

3. Système (10) selon la revendication 1 ou 2, dans lequel le système (10) comprend des moyens (52) de calcul du moment d'ordre 0 ($M^{(0)}_{sd}$) de la distribution temporelle ($M_{sd}(t)$) pour chaque couple source (14, s) - premier détecteur (16, d) et du moment d'ordre 0 équivalent ($M^{(0)}_{sd'}$) pour chaque couple source (14, s) - deuxième détecteur (22, d').

4. Système (10) selon l'une quelconque des revendications précédentes, dans lequel le système (10) comprend des moyens (52) de calcul du seul moment d'ordre 1 ($M^{(1)}_{sd}$) de la distribution temporelle ($M_{sd}(t)$) pour chaque couple source (14, s) - premier détecteur (16, d).

5. Système (10) selon les revendications 3 et 4, dans lequel le système (10) comprend un dispositif (56) de détermination, en fonction des moments d'ordre 0 ($M^{(0)}_{sd}$, $M^{(0)}_{sd'}$) et d'ordre 1 ($M^{(1)}_{sd}$) calculés, de la localisation de N fluorophore(s) (28) dans le milieu (12), N étant un nombre entier supérieur ou égal à 1, chaque premier détecteur (16, d) étant propre à recevoir un signal de fluorescence émis par le fluorophore (28) et chaque deuxième détecteur (22, d') étant propre à mesurer l'intensité ($I_{sd'}$) du signal de fluorescence émis.

6. Système (10) selon la revendication 5, dans lequel le dispositif de détermination (56) comporte des moyens (58) de réalisation d'un maillage d'un volume du milieu (12), le maillage comportant M maille(s) élémentaires, M étant un nombre entier supérieur ou égal à 1, et des moyens (60) de répartition des N fluorophore(s) (28) dans le milieu (12), chaque fluorophore (28) étant réparti dans une des M maille(s) du milieu selon une configuration (m), et des moyens (62) de calcul d'au moins un paramètre de base ($X^N_{j,m}$) combinant, pour au moins une distribution temporelle ($M_{sd}(t)$), au moins une grandeur obtenue à partir d'au moins un moment de ladite distribution et au moins une estimation de cette grandeur.

**7.** Système (10) selon l'une quelconque des revendications précédentes, dans lequel le système (10) comprend en outre une lentille (42) de focalisation des signaux émis par le milieu (12), chaque deuxième détecteur (22) étant disposé dans le plan image de la lentille (42).

**8.** Système (10) selon l'une quelconque des revendications précédentes, dans lequel le système (10) comprend en outre un filtre optique (44) agencé entre le milieu (12) et le capteur d'image (18), de manière à filtrer les signaux émis par le milieu (12) à destination du capteur d'image (18).

**9.** Système (10) selon l'une quelconque des revendications précédentes, dans lequel chaque premier détecteur (16) comporte une fibre optique (34) couplée à un module de détection résolu en temps (36).

**10.** Système (10) selon l'une quelconque des revendications précédentes, dans lequel le capteur d'image (18) est un capteur CCD ou un capteur CMOS.

**11.** Système (10) selon l'une quelconque des revendications précédentes, dans lequel les propriétés optiques comprennent au moins un élément parmi le groupe consistant en :

- les propriétés d'absorption de la lumière, caractérisées notamment par le coefficient d'absorption ($\mu_a$),
- les propriétés de diffusion, caractérisées notamment par le coefficient de diffusion réduit ($\mu'_s$) ou par le coefficient de diffusion (D), et
- les propriétés de fluorescence, caractérisées notamment par une fonction de réponse (F) d'un fluorophore, ou par une concentration (c) du fluorophore, ou encore par une grandeur fonction d'une quantité (q) du fluorophore.

**12.** Système (10) selon l'une quelconque des revendications précédentes, dans lequel le système (10) comprend des moyens de calcul d'un facteur de correction ($I_{sd'}/M^{(0)}_{sd}$) associé à la distribution temporelle ($M_{sd}(t)$) d'au moins un couple source (14, s) - premier détecteur (16, d), le facteur de correction ($I_{sd'}/M^{(0)}_{sd}$) étant fonction de l'intensité ($I_{sd'}$) mesurée par un deuxième détecteur (22, d') correspondant et du moment d'ordre 0 ($M^{(0)}_{sd}$) calculé pour ledit au moins un couple source (14, s) - premier détecteur (16, d).

**13.** Procédé de reconstruction de propriétés optiques d'un milieu diffusant (12), à l'aide d'un système de reconstruction (10) comportant au moins une source de rayonnement pulsée (14, s), au moins un premier détecteur (16, d) d'un premier type, le ou chaque premier détecteur (16, d) étant un détecteur résolu en temps, le procédé comprenant les étapes suivantes :

- l'éclairement (120) du milieu (12) par la ou chaque source de rayonnement pulsée (14, s),
- la réception, par le ou chaque premier détecteur (16, d), d'un signal émis par le milieu (12), et
- l'élaboration, pour au moins un couple source (14, s) - premier détecteur (16, d), d'une distribution temporelle $M_{sd}(t)$ du signal reçu par le premier détecteur (16, d) correspondant,
- la mesure (130) de l'intensité ($I_{sd'}$) du signal émis par le milieu (12) via l'acquisition d'une image du milieu à l'aide d'au moins un deuxième détecteur (22, d') d'un deuxième type distinct du premier type, ladite intensité ($I_{sd'}$) correspondant à un moment d'ordre 0 équivalent ($M^{(0)}_{sd'}$) pour le couple source (14, s) - deuxième détecteur (22, d') correspondant, le ou chaque deuxième détecteur (22, d') étant inclus dans le système de reconstruction (10) et formé d'un ensemble de pixel(s) d'un capteur d'image (18),

**caractérisé en ce qu'**il comprend en outre les étapes suivantes:

- le calcul d'au moins un paramètre de base ($X^N_{j,m}$) en combinant une grandeur caractéristique de la distribution temporelle ($M_{sd}(t)$) correspondant à au moins un couple source (14, s) - premier détecteur (16, d) et l'intensité ($I_{sd'}$) mesurée pour au moins un couple source (14, s) - deuxième détecteur (22, d'), et
- la reconstruction des propriétés optiques à partir du paramètre de base ($X^N_{j,m}$) calculé.

**14.** Procédé selon la revendication 13, dans lequel le procédé comprend en outre le calcul du moment d'ordre 0 ($M^{(0)}_{sd}$) de la distribution temporelle ($M_{sd}(t)$) pour chaque couple source (14, s) - premier détecteur (16, d) et du moment d'ordre 0 équivalent ($M^{(0)}_{sd'}$) pour chaque couple source (14, s) - deuxième détecteur (22, d').

**Patentansprüche**

**1.** System (10) zum Rekonstruieren von optischen Eigenschaften eines streuenden Mediums (12), wobei das System zum Rekonstruieren (10) aufweist:

- mindestens eine gepulste Strahlungsquelle (14, s), die in der Lage ist, das streuende Medium (12) zu beleuchten,
- mindestens einen ersten Detektor (16, d) eines ersten Typs, der in der Lage ist, ein von dem Medium (12) emittiertes Signal zu empfangen, wobei der oder jeder erste Detektor (16, d) ein zeitaufgelöster Detektor ist,
- Mittel zum Erstellen, für mindestens ein Quelle- (14, s) -erster Detektor- (16, d) - Paar, einer zeitlichen Verteilung ($M_{sd}(t)$) des von dem korrespondierenden ersten Detektor (16, d) empfangenen Signals,

wobei das System (10) ferner mindestens einen zweiten Detektor (22, d') eines zweiten Typs aufweist, der sich von dem ersten Typ unterscheidet, wobei der oder jeder zweite Detektor (22, d') aus einem Satz von Pixeln eines Bildsensors (18) gebildet ist, der in der Lage ist, ein Bild des Mediums (12) zu erfassen, wobei der oder jeder zweite Detektor (22, d') in der Lage ist, eine Stärke ($I_{sd'}$) des von dem Medium (12) emittierten Signals zu messen, wobei die Stärke ($I_{sd'}$) für das entsprechende Quelle- (14, s) -zweiter Detektor- (22, d') -Paar einem äquivalenten Moment nullter Ordnung ($M^{(0)}_{sd'}$) entspricht, wobei das System (10) **dadurch gekennzeichnet ist, dass** es ferner Mittel (62) aufweist zum Berechnen mindestens eines Basisparameters ($X^N_{j,m}$) durch Kombinieren einer Größe, die für die zeitliche Verteilung ($M_{sd}(t)$) charakteristisch ist, die mindestens einem Quelle-(14, s) -erster Detektor- (16, d) -Paar entspricht, und der Stärke ($I_{Sd'}$), die für mindestens ein Quelle- (14, s) -zweiter Detektor- (22, d') -Paar gemessen wird, wobei das Rekonstruieren der optischen Eigenschaften ausgehend von dem berechneten Basisparameter ($X^N_{j,m}$) erfolgt.

**2.** System (10) gemäß Anspruch 1, wobei der oder jeder zweite Detektor (22, d') ein nicht-zeitaufgelöster Detektor ist.

**3.** System (10) gemäß Anspruch 1 oder 2, wobei das System (10) Mittel (52) aufweist zum Berechnen des Moments nullter Ordnung ($M^{(0)}_{sd}$) der zeitlichen Verteilung ($M_{sd}(t)$) für jedes Quelle- (14, s) -erster Detektor- (16, d) -Paar und des äquivalenten Moments nullter Ordnung ($M^{(0)}_{sd'}$) für jedes Quelle- (14, s) -zweiter Detektor- (22, d') -Paar.

**4.** System (10) gemäß irgendeinem der vorhergehenden Ansprüche, wobei das System (10) Mittel (52) aufweist zum Berechnen des einzigen Moments erster Ordnung ($M^{(1)}_{sd}$) der zeitlichen Verteilung ($M_{sd}(t)$) für jedes Quelle- (14, s) - erster Detektor- (16, d) -Paar.

**5.** System (10) gemäß den Ansprüchen 3 und 4, wobei das System (10) aufweist eine Vorrichtung (56) zum Ermitteln, in Abhängigkeit von den berechneten Momenten nullter Ordnung ($M^{(0)}_{sd}$, $M^{(0)}_{sd'}$) und erster Ordnung ($M^{(1)}_{sd}$), der Lage von N Fluorophor(en) (28) in dem Medium (12), wobei N eine ganze Zahl ist, die größer oder gleich 1 ist, wobei jeder erste Detektor (16, d) in der Lage ist, ein Fluoreszenzsignal zu empfangen, das von dem Fluorophor (28) emittiert wird, und jeder zweite Detektor (22, d') in der Lage ist, die Stärke ($I_{sd'}$) des emittierten Fluoreszenzsignals zu messen.

**6.** System (10) gemäß Anspruch 5, wobei die Vorrichtung zum Ermitteln (56) aufweist Mittel (58) zum Realisieren einer Vernetzung eines Volumens des Mediums (12), wobei die Vernetzung M elementare Netze aufweist, wobei M eine ganze Zahl ist, die größer oder gleich 1 ist, und Mittel (60) zum Verteilen der N Fluorophor(e) (28) in dem Medium (12), wobei jeder Fluorophor (28) in einem der M Netze des Mediums gemäß einer Konfiguration (m) verteilt ist, und Mittel (62) zum Berechnen mindestens eines Basisparameters ($X^N_{j,m}$), der für mindestens eine zeitliche Verteilung ($M_{sd}(t)$) mindestens eine Größe, die ausgehend von mindestens einem Moment der Verteilung erhalten wird, und mindestens eine Schätzung dieser Größe kombiniert.

**7.** System (10) gemäß irgendeinem der vorhergehenden Ansprüche, wobei das System (10) ferner aufweist eine Linse (42) zum Bündeln der Signale, die von dem Medium (12) ausgegeben werden, wobei jeder zweite Detektor (22) in der Bildebene der Linse (42) angeordnet ist.

8. System (10) gemäß irgendeinem der vorhergehenden Ansprüche, wobei das System (10) ferner einen optischen Filter (44) aufweist, der zwischen dem Medium (12) und dem Bilddetektor (18) angeordnet ist, um die Signale zu filtern, die von dem Medium (12) an den Bildsensor (18) emittiert werden.

9. System (10) gemäß irgendeinem der vorhergehenden Ansprüche, wobei jeder erste Detektor (16) eine optische Faser (34) aufweist, die mit einem zeitaufgelösten Detektionsmodul (36) verbunden ist.

10. System (10) gemäß irgendeinem der vorhergehenden Ansprüche, wobei der Bildsensor (18) ein CCD-Sensor oder ein CMOS-Sensor ist.

11. System (10) gemäß irgendeinem der vorhergehenden Ansprüche, wobei die optischen Eigenschaften mindestens ein Element aus der Gruppe aufweisen, die aus folgendem besteht:

- den Lichtabsorptionseigenschaften, die insbesondere durch den Absorptionskoeffizienten ($\mu_a$) gekennzeichnet sind,
- den Diffusionseigenschaften, die insbesondere durch den reduzierten Diffusionskoeffizienten ($\mu'_s$) oder durch den Diffusionskoeffizienten (D) gekennzeichnet sind, und
- den Fluoreszenzeigenschaften, die insbesondere durch eine Antwortfunktion (F) eines Fluorophors oder durch eine Konzentration (c) des Fluorophors oder aber durch eine Größe gekennzeichnet sind, die von einer Menge (q) des Fluorophors abhängig ist.

12. System (10) gemäß irgendeinem der vorhergehenden Ansprüche, wobei das System (10) Mittel aufweist zum Berechnen eines Korrekturfaktors ($I_{sd'}/M^{(0)}_{sd}$), der mit der zeitlichen Verteilung ($M_{sd}(t)$) mindestens eines Quelle- (14, s) -erster Detektor- (16, d) -Paares verknüpft ist, wobei der Korrekturfaktor ($I_{sd'}/M^{(0)}_{sd}$) abhängig ist von der Stärke ($I_{sd'}$) die von einem korrespondierenden zweiten Detektor (22, d') gemessen wird, und dem Moment nullter Ordnung ($M^{(0)}_{sd}$), der für das mindestens eine Quelle- (14, s) -erster Detektor-(16, d) -Paar berechnet wird.

13. Verfahren zum Rekonstruieren von optischen Eigenschaften eines streuenden Mediums (12) mit Hilfe eines Systems zum Rekonstruieren (10), aufweisend mindestens eine gepulste Strahlungsquelle (14, s), mindestens einen ersten Detektor (16, d) eines ersten Typs, wobei der oder jeder erste Detektor (16, d) ein zeitaufgelöster Detektor ist, wobei das Verfahren die folgenden Schritte aufweist:

- Beleuchten (120) des Mediums (12) durch die oder jede gepulste Strahlungsquelle (14, s),
- Empfangen, durch den oder jeden ersten Detektor (16, d) eines von dem Medium (12) emittierten Signals, und
- Erstellen, für mindestens ein Quelle- (14, s) - erster Detektor- (16, d) -Paar einer zeitlichen Verteilung ($M_{sd}(t)$) des von dem korrespondierenden ersten Detektor (16, d) empfangenen Signals,
- Messen (130) der Stärke ($I_{sd'}$) des von dem Medium (12) emittierten Signals über das Erfassen eines Bildes des Mediums mit Hilfe mindestens eines zweiten Detektors (22, d') eines zweiten Typs, der sich von dem ersten Typ unterscheidet, wobei die Stärke ($I_{sd'}$) einem äquivalenten Moment nullter Ordnung ($M^{(0)}_{sd'}$) für das korrespondierende Quelle- (14, s) -zweiter Detektor- (22, d') -Paar entspricht, wobei der oder jeder zweite Detektor (22, d') in dem System zum Rekonstruieren (10) enthalten ist und aus einem Satz von Pixeln eines Bildsensors (18) gebildet ist,

**dadurch gekennzeichnet, dass** es ferner die folgenden Schritte aufweist:

- Berechnen mindestens eines Basisparameters ($X^N_{j,m}$) durch Kombinieren einer Größe, die für die zeitliche Verteilung ($M_{sd}(t)$) charakteristisch ist, die mindestens einem Quelle- (14, s) -erster Detektor- (16, d) -Paar entspricht, und der Stärke ($I_{sd'}$), die für mindestens ein Quelle- (14, s) - zweiter Detektor- (22, d') -Paar gemessen wird, und
- Rekonstruieren der optischen Eigenschaften ausgehend von dem berechneten Basisparameter ($X^N_{j,m}$).

14. Verfahren gemäß Anspruch 13, wobei das Verfahren ferner aufweist das Berechnen des Moments nullter Ordnung ($M^{(0)}_{sd}$) der zeitlichen Verteilung ($M_{sd}(t)$) für jedes Quelle-(14, s) -erster Detektor- (16, d) -Paar und des äquivalenten Moments nullter Ordnung ($M^{(0)}_{sd'}$) für jedes Quelle- (14, s) - zweiter Detektor- (22, d') -Paar.

**Claims**

1. A system (10) for reconstructing optical properties of a diffusing medium (12), the reconstruction system (10) comprising:

   - at least one pulsed radiation source (14, s) capable of illuminating the diffusing medium (12),
   - at least one first detector (16, d) of a first type, capable of receiving a signal emitted by the medium (12), the or each first detector (16, d) being a time-resolved detector,
   - means for processing, for at least one source (14, s) - first detector (16, d) pair, a time distribution ($M_{sd}(t)$) of the signal received by the corresponding first detector (16, d),

   the system (10) further comprising at least one second detector (22, d') of a second type distinct from the first type, the or each second detector (22, d') being made up of a set of pixel(s) from an image sensor (18) capable of acquiring an image of the medium (12), the or each second detector (22, d') being capable of measuring an intensity ($I_{sd'}$) of the signal emitted by the medium (12), said intensity ($I_{sd'}$) corresponding to an equivalent moment of order 0 ($M^{(0)}_{sd'}$) for the corresponding source (14, s) - second detector (22, d') pair,
   the system (10) being **characterized in that** it further comprises means (62) for computing at least one base parameter ($X^N_{j,m}$), by combining a magnitude that is characteristic of the time distribution ($M_{sd}(t)$) corresponding to at least one source (14, s) - first detector (16, d) pair with the intensity ($I_{sd'}$) measured for at least one source (14, s) - second detector (22, d') pair, the optical properties being reconstructed from the computed base parameter ($X^N_{j,m}$).

2. The system (10) according to claim 1, wherein the or each second detector (22, d') is a non-time-resolved detector.

3. The system (10) according to claim 1 or 2, wherein the system (10) comprises means (52) for computing the moment of order 0 ($M^{(0)}_{sd}$) of the time distribution ($M_{sd}(t)$) for each source (14, s) - first detector (16, d) pair and the equivalent moment of order 0 ($M^{(0)}_{sd'}$) for each source (14, s) - second detector (22, d') pair.

4. The system (10) according to any one of the preceding claims, wherein the system (10) comprises means (52) for computing a single moment of order 1 ($M^{(1)}_{sd}$) of the time distribution ($M_{sd}(t)$) for each source (14, s) - first distribution (16, d) pair.

5. The system (10) according to claims 3 and 4, wherein the system (10) comprises a device (56) for determining, as a function of the computed moments of order 0 ($M^{(0)}_{sd}$, $M^{(0)}_{sd'}$) and order 1 ($M^{(1)}_{sd}$), the localization of N fluorophore(s) (28) in the medium (12), N being an integer greater than or equal to 1, each first detector (16, d) being capable of receiving a fluorescence signal emitted by the fluorophore (28) and each second detector (22, d') being capable of measuring the intensity ($I_{sd'}$) of the emitted fluorescence signal.

6. The system (10) according to claim 5, wherein the determination device (56) includes means (58) for producing a mesh of a volume of the medium (12), the mesh including M elementary link(s), M being an integer greater than or equal to 1, and means (60) for distributing N fluorophore(s) (28) in the medium (12), each fluorophore (28) being distributed in one of the M link(s) of the medium according to a configuration (m), and means (62) for computing at least one base parameter ($X^N_{j,m}$), the or each base parameter ($X^N_{j,m}$) combining, for at least one time distribution ($M_{sd}(t)$), at least one magnitude obtained from at least one moment of said distribution and at least one estimate of that magnitude.

7. The system (10) according to any one of the preceding claims, wherein the system (10) also comprises a lens (42) for focusing the signals emitted by the medium (12), each second detector (22) being positioned in the image plane of the lens (42).

8. The system (10) according to any one of the preceding claims, wherein the system (10) also comprises an optical filter (44) arranged between the medium (12) and the image sensor (18), so as to filter the signals emitted by the medium (12) to the image sensor (18).

9. The system (10) according to any one of the preceding claims, wherein each first detector (16) includes an optical fiber (34) coupled to a time-resolved detection module (36).

10. The system (10) according to any one of the preceding claims, wherein the image sensor (18) is a CCD sensor or a CMOS sensor.

**11.** The system (10) according to any one of the preceding claims, wherein the optical properties comprise at least one element from among the following group:

- light absorption properties, **characterized in** particular by the absorption coefficient ($\mu_a$),
- diffusion properties, **characterized in** particular by the reduced diffusion coefficient ($\mu'_s$) or the diffusion coefficient (D), and
- fluorescence properties, in particular **characterized by** a response function (F) of a fluorophore, or by a concentration (c) of the fluorophore, or by a magnitude depending on a quantity (q) of the fluorophore.

**12.** The system (10) according to any one of the preceding claims, wherein the system (10) comprises means for computing a correction factor ($I_{sd'}/M^{(0)}_{sd'}$) associated with the time distribution ($M_{sd}(t)$) of at least one source (14, s) - first detector (16, d) pair, the correction factor ($I_{sd'}/M^{(0)}_{sd'}$) depending on the intensity ($I_{sd'}$) measured by a second corresponding detector (22, d') and the moment of order 0 ($M^{(0)}_{sd}$) computed for said at least one source (14, s) - first detector (16, d) pair.

**13.** A method for reconstructing optical properties of a diffusing medium (12), using a reconstruction system (10) including at least one pulsed radiation source (14, s), at least one first detector (16, d) of a first type, the or each first detector (16, d) being a time-resolved detector,
the method comprising the following steps:

- lighting (120) the medium (12) using the or each pulsed radiation source (14, s),
- reception, by the or each first detector (16, d), of a signal emitted by the medium (12), and
- processing, for at least one source (14, s) - first detector (16, d) pair, a time distribution $M_{sd}(t)$ of the signal received by the corresponding first detector (16, d),
- measuring (130) the intensity ($I_{sd'}$) of the signal emitted by the medium (12) by acquiring an image of the medium using at least one second detector (22, d') of a second type distinct from the first type, said intensity ($I_{sd'}$) corresponding to an equivalent moment of order 0 ($M^{(0)}_{sd}$) for the corresponding source (14, s) - second detector (22, d') pair, the or each second detector (22, d') being included in the reconstruction system (10) and made up of a set of pixel(s) from an image sensor (18),

**characterized in that** it further comprises the following steps:

- computing at least one base parameter ($X^N_{j,m}$), by combining a magnitude that is characteristic of the time distribution ($M_{sd}(t)$) corresponding to at least one source (14, s) - first detector (16, d) pair with the intensity ($I_{sd'}$) measured for at least one source (14, s) - second detector (22, d') pair, and
- reconstructing the optical properties from the computed base parameter ($X^N_{j,m}$).

**14.** The method according to claim 13, wherein the method also comprises computing the moment of order 0 ($M^{(0)}_{sd}$) of the time distribution ($M_{sd}(t)$) for each source (14, s) - first detector (16, d) pair and the equivalent moment of order 0 ($M^{(0)}_{sd}$) for each source (14, s) - second detector (22, d') pair.

## FIG.1

_FIG.2_

_FIG.3_

Calibrage préliminaire du dispositif

100

Identification de la zone à analyser à l'aide du capteur d'image

110

Eclairement de la zone à analyser à l'aide de la source de rayonnement pulsée

120

Mesure de l'intensité lumineuse en une pluralité de points de la zone à analyser à l'aide du capteur d'image et mesure de la distribution temporelle des photons à l'aide du module de detection résolu en temps

130

Calcul des coefficients d'absorption $\mu_a$ et de diffusion $\mu'_s$

140

Localisation d'au moins un fluorophore dans la zone à analyser

150

FIG.4

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 2302362 A1
- WO 2005043138 A1
- WO 2009046985 A2
- EP 2182343 A1

**Littérature non-brevet citée dans la description**

- **STEINVALL et al.** High resolution ladar using time-correlated single-photon counting. *SPIE,* Janvier 2008, 6950